# EUROPEAN PATENT APPLICATION

(11) **EP 1 820 511 A1**
(43) Date of publication of application: **22.08.2007**
(21) Application number: 07101564.8
(22) Date of filing: 01.02.2007
(51) Int. Cl.: A61K 39/145

(54) **MHC binding peptides and their uses**

(30) Priority: 20.02.2006 GB 0603336; 24.04.2006 GB 0608138; 19.05.2006 GB 0609896
(71) Applicant: Prolmmune Limited, Oxford, Oxfordshire OX4 4GA (GB)
(72) Inventor: Napper, Catherine Elizabeth, Witney OX28 3UD (GB); Schwabe, Nikolai Franz Gregor, Oxford OX2 6BP (GB)
(74) Representative: Cremer & Cremer

(57) **Abstract**

The present invention is concerned with MHC binding peptides derived from the avian influenza virus H5N1 and in particular MHC class I restricted binding peptides from the Haemagglutinin 5 (H5) protein from this virus and their uses.

SEQUENCE LISTING

<110>  ProImmune Limited

<120>  MHC Binding Peptides And Their Uses

<130>  PRO 07008 EP

<150>  GB0603336.9
<151>  2006-02-20

<150>  GB0608138.4
<151>  2006-04-24

<150>  GB0609896.6
<151>  2006-05-19

<160>  43

<170>  PatentIn version 3.3

<210>  1
<211>  568
<212>  PRT
<213>  H5N1

<400>  1

Met Glu Lys Ile Val Leu Leu Phe Ala Ile Val Ser Leu Val Lys Ser
1               5                   10                  15

Asp Gln Ile Cys Ile Gly Tyr His Ala Asn Asn Ser Thr Glu Gln Val
            20                  25                  30

Asp Thr Ile Met Glu Lys Asn Val Thr Val Thr His Ala Gln Asp Ile
        35                  40                  45

Leu Glu Lys Lys His Asn Gly Lys Leu Cys Asp Leu Asp Gly Val Lys
    50                  55                  60

Pro Leu Ile Leu Arg Asp Cys Ser Val Ala Gly Trp Leu Leu Gly Asn
65                  70                  75                  80

Pro Met Cys Asp Glu Phe Ile Asn Val Pro Glu Trp Ser Tyr Ile Val
                85                  90                  95

Glu Lys Ala Asn Pro Val Asn Asp Leu Cys Tyr Pro Gly Asp Phe Asn
            100                 105                 110

Asp Tyr Glu Glu Leu Lys His Leu Leu Ser Arg Ile Asn His Phe Glu
        115                 120                 125

Lys Ile Gln Ile Ile Pro Lys Ser Ser Trp Ser Ser His Glu Ala Ser
    130                 135                 140

Leu Gly Val Ser Ser Ala Cys Pro Tyr Gln Gly Lys Ser Ser Phe Phe
145                 150                 155                 160

Arg Asn Val Val Trp Leu Ile Lys Lys Asn Ser Thr Tyr Pro Thr Ile
                165                 170                 175

Lys Arg Ser Tyr Asn Asn Thr Asn Gln Glu Asp Leu Leu Val Leu Trp
            180                 185                 190

Gly Ile His His Pro Asn Asp Ala Ala Glu Gln Thr Lys Leu Tyr Gln
        195                 200                 205

Asn Pro Thr Thr Tyr Ile Ser Val Gly Thr Ser Thr Leu Asn Gln Arg
    210                 215                 220

Leu Val Pro Arg Ile Ala Thr Arg Ser Lys Val Asn Gly Gln Ser Gly
225                 230                 235                 240

Arg Met Glu Phe Phe Trp Thr Ile Leu Lys Pro Asn Asp Ala Ile Asn
                245                 250                 255

Phe Glu Ser Asn Gly Asn Phe Ile Ala Pro Glu Tyr Ala Tyr Lys Ile
            260                 265                 270

Val Lys Lys Gly Asp Ser Thr Ile Met Lys Ser Glu Leu Glu Tyr Gly
        275                 280                 285

Asn Cys Asn Thr Lys Cys Gln Thr Pro Met Gly Ala Ile Asn Ser Ser
    290                 295                 300

Met Pro Phe His Asn Ile His Pro Leu Thr Ile Gly Glu Cys Pro Lys
305                 310                 315                 320

Tyr Val Lys Ser Asn Arg Leu Val Leu Ala Thr Gly Leu Arg Asn Ser
                325                 330                 335

Pro Gln Arg Glu Arg Arg Arg Lys Lys Arg Gly Leu Phe Gly Ala Ile
            340                 345                 350

Ala Gly Phe Ile Glu Gly Gly Trp Gln Gly Met Val Asp Gly Trp Tyr
        355                 360                 365

Gly Tyr His His Ser Asn Glu Gln Gly Ser Gly Tyr Ala Ala Asp Lys
    370                 375                 380

Glu Ser Thr Gln Lys Ala Ile Asp Gly Val Thr Asn Lys Val Asn Ser
385                 390                 395                 400

Ile Ile Asp Lys Met Asn Thr Gln Phe Glu Ala Val Gly Arg Glu Phe
                405                 410                 415

Asn Asn Leu Glu Arg Arg Ile Glu Asn Leu Asn Lys Lys Met Glu Asp
            420                 425                 430

Gly Phe Leu Asp Val Trp Thr Tyr Asn Ala Glu Leu Leu Val Leu Met
        435                 440                 445

Glu Asn Glu Arg Thr Leu Asp Phe His Asp Ser Asn Val Lys Asn Leu
    450                 455                 460

Tyr Asp Lys Val Arg Leu Gln Leu Arg Asp Asn Ala Lys Glu Leu Gly
465                 470                 475                 480

Asn Gly Cys Phe Glu Phe Tyr His Lys Cys Asp Asn Glu Cys Met Glu
                485                 490                 495

Ser Val Arg Asn Gly Thr Tyr Asp Tyr Pro Gln Tyr Ser Glu Glu Ala
            500                 505                 510

Arg Leu Lys Arg Glu Glu Ile Ser Gly Val Lys Leu Glu Ser Ile Gly
        515                 520                 525

Ile Tyr Gln Ile Leu Ser Ile Tyr Ser Thr Val Ala Ser Ser Leu Ala
    530                 535                 540

Leu Ala Ile Met Val Ala Gly Leu Ser Leu Trp Met Cys Ser Asn Gly
545                 550                 555                 560

Ser Leu Gln Cys Arg Ile Cys Ile
                565

<210>  2
<211>  10
<212>  PRT
<213>  Artificial Sequence

<220>
<223>  T cell epitope

<400>  2

Tyr Ser Glu His Pro Phe Thr Ser Gln Tyr
1               5                   10

<210>  3
<211>  9
<212>  PRT

<213>  Artificial Sequence

<220>
<223>  T cell epitope

<400>  3

Ala Ala Gly Ile Gly Ile Leu Thr Val
1               5


<210>  4
<211>  9
<212>  PRT
<213>  Artificial Sequence

<220>
<223>  T cell epitope

<400>  4

Gly Ile Leu Gly Phe Val Phe Thr Leu
1               5


<210>  5
<211>  9
<212>  PRT
<213>  Artificial Sequence

<220>
<223>  T cell epitope

<400>  5

Ile Leu Arg Gly Ser Val Ala His Lys
1               5


<210>  6
<211>  10
<212>  PRT
<213>  Artificial Sequence

<220>
<223>  T cell epitope

<400>  6

Thr Pro Arg Val Thr Gly Gly Gly Ala Met
1               5                   10


<210>  7
<211>  8
<212>  PRT
<213>  Artificial Sequence

<220>
<223>  T cell epitope

<400>  7

Arg Ala Lys Phe Lys Gln Leu Leu
1               5


<210>  8
<211>  11
<212>  PRT
<213>  Artificial Sequence

<220>
<223>  T cell epitope

<400>  8

His Pro Val Gly Glu Ala Asp Tyr Phe Glu Tyr
1               5                   10


<210>  9
<211>  9
<212>  PRT
<213>  Artificial Sequence

<220>
<223>  9-mer peptide from the H5 protein in H5N1

<400>  9

Lys Ser Asp Gln Ile Cys Ile Gly Tyr
1               5


<210>  10
<211>  9
<212>  PRT
<213>  Artificial Sequence

<220>
<223>  9-mer peptide from the H5 protein in H5N1

<400>  10

Phe Ile Asn Val Pro Glu Trp Ser Tyr
1               5


<210>  11
<211>  9
<212>  PRT
<213>  Artificial Sequence

<220>
<223>  9-mer peptide from the H5 protein in H5N1

<400>  11

Val Leu Leu Phe Ala Ile Val Ser Leu
1               5


<210>  12
<211>  9
<212>  PRT

<213>  Artificial Sequence

<220>
<223>  9-mer peptide from the H5 protein in H5N1

<400>  12

Leu Leu Phe Ala Ile Val Ser Leu Val
1               5


<210>  13
<211>  9
<212>  PRT
<213>  Artificial Sequence

<220>
<223>  9-mer peptide from the H5 protein in H5N1

<400>  13

Phe Ala Ile Val Ser Leu Val Lys Ser
1               5


<210>  14
<211>  9
<212>  PRT
<213>  Artificial Sequence

<220>
<223>  9-mer peptide from the H5 protein in H5N1

<400>  14

Ala Ile Val Ser Leu Val Lys Ser Asp
1               5


<210>  15
<211>  9
<212>  PRT
<213>  Artificial Sequence

<220>
<223>  9-mer peptide from the H5 protein in H5N1

<400>  15

Ile Val Ser Leu Val Lys Ser Asp Gln
1               5


<210>  16
<211>  9
<212>  PRT
<213>  Artificial Sequence

<220>
<223>  9-mer peptide from the H5 protein in H5N1

<400>  16

Ser Leu Val Lys Ser Asp Gln Ile Cys
1               5


<210>  17
<211>  9
<212>  PRT
<213>  Artificial Sequence

<220>
<223>  9-mer peptide from the H5 protein in H5N1

<400>  17

His Ala Asn Asn Ser Thr Glu Gln Val
1               5


<210>  18
<211>  9
<212>  PRT
<213>  Artificial Sequence

<220>
<223>  9-mer peptide from the H5 protein in H5N1

<400>  18

Thr Ile Met Glu Lys Asn Val Thr Val
1               5


<210>  19
<211>  9
<212>  PRT
<213>  Artificial Sequence

<220>
<223>  9-mer peptide from the H5 protein in H5N1

<400>  19

Ile Met Glu Lys Asn Val Thr Val Thr
1               5


<210>  20
<211>  9
<212>  PRT
<213>  Artificial Sequence

<220>
<223>  9-mer peptide from the H5 protein in H5N1

<400>  20

Met Glu Lys Asn Val Thr Val Thr His
1               5


<210>  21
<211>  9
<212>  PRT

<213>  Artificial Sequence

<220>
<223>  9-mer peptide from the H5 protein in H5N1

<400>  21

Ala Gln Asp Ile Leu Glu Lys Lys His
1               5


<210>  22
<211>  9
<212>  PRT
<213>  Artificial Sequence

<220>
<223>  9-mer peptide from the H5 protein in H5N1

<400>  22

Lys Leu Cys Asp Leu Asp Gly Val Lys
1               5


<210>  23
<211>  9
<212>  PRT
<213>  Artificial Sequence

<220>
<223>  9-mer peptide from the H5 protein in H5N1

<400>  23

Leu Ile Leu Arg Asp Cys Ser Val Ala
1               5


<210>  24
<211>  9
<212>  PRT
<213>  Artificial Sequence

<220>
<223>  9-mer peptide from the H5 protein in H5N1

<400>  24

Pro Met Cys Asp Glu Phe Ile Asn Val
1               5


<210>  25
<211>  9
<212>  PRT
<213>  Artificial Sequence

<220>
<223>  9-mer peptide from the H5 protein in H5N1

<400>  25

Asn Val Pro Glu Trp Ser Tyr Ile Val
1               5


<210>  26
<211>  9
<212>  PRT
<213>  Artificial Sequence

<220>
<223>  9-mer peptide from the H5 protein in H5N1

<400>  26

Tyr Ile Val Glu Lys Ala Asn Pro Val
1               5


<210>  27
<211>  9
<212>  PRT
<213>  Artificial Sequence

<220>
<223>  9-mer peptide from the H5 protein in H5N1

<400>  27

Asn Val Thr Val Thr His Ala Gln Asp
1               5


<210>  28
<211>  9
<212>  PRT
<213>  Artificial Sequence

<220>
<223>  9-mer peptide from the H5 protein in H5N1

<400>  28

Ile Leu Glu Lys Lys His Asn Gly Lys
1               5


<210>  29
<211>  9
<212>  PRT
<213>  Artificial Sequence

<220>
<223>  9-mer peptide from the H5 protein in H5N1

<400>  29

Leu Val Lys Ser Asp Gln Ile Cys Ile
1               5


<210>  30
<211>  9
<212>  PRT

<213>  Artificial Sequence

<220>
<223>  9-mer peptide from the H5 protein in H5N1

<400>  30

Ile Leu Arg Asp Cys Ser Val Ala Gly
1               5


<210>  31
<211>  9
<212>  PRT
<213>  Artificial Sequence

<220>
<223>  9-mer peptide from the H5 protein in H5N1

<400>  31

Val Ser Leu Val Lys Ser Asp Gln Ile
1               5


<210>  32
<211>  9
<212>  PRT
<213>  Artificial Sequence

<220>
<223>  9-mer peptide from the H5 protein in H5N1

<400>  32

Asp Thr Ile Met Glu Lys Asn Val Thr
1               5


<210>  33
<211>  9
<212>  PRT
<213>  Artificial Sequence

<220>
<223>  9-mer peptide from the H5 protein in H5N1

<400>  33

Val Thr His Ala Gln Asp Ile Leu Glu
1               5


<210>  34
<211>  9
<212>  PRT
<213>  Artificial Sequence

<220>
<223>  9-mer peptide from the H5 protein in H5N1

<400>  34

Asp Ile Leu Glu Lys Lys His Asn Gly
1               5


<210>  35
<211>  9
<212>  PRT
<213>  Artificial Sequence

<220>
<223>  9-mer peptide from the H5 protein in H5N1

<400>  35

Lys Pro Leu Ile Leu Arg Asp Cys Ser
1               5


<210>  36
<211>  9
<212>  PRT
<213>  Artificial Sequence

<220>
<223>  9-mer peptide from the H5 protein in H5N1

<400>  36

Pro Leu Ile Leu Arg Asp Cys Ser Val
1               5


<210>  37
<211>  9
<212>  PRT
<213>  Artificial Sequence

<220>
<223>  9-mer peptide from the H5 protein in H5N1

<400>  37

Val Ala Gly Trp Leu Leu Gly Asn Pro
1               5


<210>  38
<211>  9
<212>  PRT
<213>  Artificial Sequence

<220>
<223>  9-mer peptide from the H5 protein in H5N1

<400>  38

Trp Leu Leu Gly Asn Pro Met Cys Asp
1               5


<210>  39
<211>  9
<212>  PRT

<213>  Artificial Sequence

<220>
<223>  9-mer peptide from the H5 protein in H5N1

<400>  39

Gly Asn Pro Met Cys Asp Glu Phe Ile
1               5


<210>  40
<211>  9
<212>  PRT
<213>  Artificial Sequence

<220>
<223>  9-mer peptide from the H5 protein in H5N1

<400>  40

Leu Phe Ala Ile Val Ser Leu Val Lys
1               5


<210>  41
<211>  9
<212>  PRT
<213>  Artificial Sequence

<220>
<223>  9-mer peptide from the H5 protein in H5N1

<400>  41

Lys Lys His Asn Gly Lys Leu Cys Asp
1               5


<210>  42
<211>  9
<212>  PRT
<213>  Artificial Sequence

<220>
<223>  9-mer peptide from the H5 protein in H5N1

<400>  42

Ala Gly Trp Leu Leu Gly Asn Pro Met
1               5


<210>  43
<211>  9
<212>  PRT
<213>  Artificial Sequence

<220>
<223>  9-mer peptide from the H5 protein in H5N1

<400>  43

Leu Gly Asn Pro Met Cys Asp Glu Phe
1               5

## Description

### Introduction

The present invention is concerned with MHC binding peptides derived from the avian influenza virus H5N1 and in particular MHC class I restricted binding peptides from the Haemagglutinin 5 (H5) protein from this virus and their uses.

Major Histocompatibility Complex (MHC) molecules, which are found on the cell surface in tissues, play an important role in presenting cellular antigens in the form of short linear peptides to T cells by interacting with T cell receptors (TCRs) present on the surface of T cells.

In the above interaction of the MHC molecule and the T cell, the T cell recognizes and binds to the peptide bound in the MHC binding groove and the surrounding area of the MHC binding groove itself. In order for a peptide to bind to an MHC molecule and therefore be able to cause an immune response *in vivo* it needs to fit the properties of the binding groove, which are also referred to as the "binding motif", of the relevant MHC allele in question. This binding motif restricts the type of peptides that can bind to a given MHC allele. Binding peptides matching the binding motif of a given MHC allele are also referred to as "restricted" to that allele.

It is known that class I MHC molecules typically bind peptide fragments of 8 to 11 amino acid length in their binding groove formed between the MHC class I alpha1 and alpha2 domains. For Class II MHC molecules binding of the peptides occurs in the groove formed between the alpha1 and beta1 domains of the molecule, the length of binding peptide varies more widely, and these peptides are typically 15 to 25 amino acids long or even longer, e.g. up to 30 amino acids length.

Understanding which peptides in a protein sequence can bind to a given MHC molecule and cause a T cell immune response is of considerable interest for understanding cellular immunity and in order to design and monitor the effectiveness of immunotherapeutic products, such as vaccines. MHC binding peptides that cause T cell immune responses are also referred to as "T cell epitopes".

The relevance of identifying MHC-binding peptides has also been described extensively in the literature, such as in US 5,585,461 or in US 6,733,973, which describe the discovery and uses of MHC class I restricted T cell epitopes for the tumour antigen MAGE-3 and cytomegalovirus (CMV), respectively. US 5,585,461 and US 6,733,973 are incorporated herein by reference.

The H5N1 strain of avian influenza has been recognized as a serious threat to human health by the World Health Organisation. H5N1 avian influenza usually only affects birds, but it is thought to have the ability to mutate and acquire genes from viruses infecting other mammalian species. It can infect humans who have almost no natural immunity to the virus with high mortality. If the H5N1 virus mutates to increase its infectiousness to humans, a severe global flu pandemic could ensue. Presently no vaccine is available to treat H5N1 influenza in humans. Understanding immune responses to avian influenza is therefore critical in order to develop effective human therapies in the future.

### The Invention

We have used an MHC-peptide binding assay as described in our co-pending patent application GB0519029.3, which is incorporated herein by reference, to identify MHC-binding peptides from the avian influenza virus H5N1. To perform our binding assay we synthesized a library of overlapping 9-mer peptides, offset from one another by one amino acid for the first 102 amino acids of the H5 protein from H5N1 Genbank / Swissprot ID: A/Vietnam/1203/04, Accession No.: AAW80717:

SEQ ID NO: 1:
MEKIVLLFAI VSLVKSDQIC IGYHANNSTE QVDTIMEKNV TVTHAQDILE KKHNGKLCDL 60
DGVKPLILRD CSVAGWLLGN PMCDEFINVP EWSYIVEKAN PVNDLCYPGD FNDYEELKHL 120
LSRINHFEKI QIIPKSSWSS HEASLGVSSA CPYQGKSSFF RNVVWLIKKN STYPTIKRSY 180
NNTNQEDLLV LWGIHHPNDA AEQTKLYQNP TTYISVGTST LNQRLVPRIA TRSKVNGQSG 240
RMEFFWTILK PNDAINFESN GNFIAPEYAY KIVKKGDSTI MKSELEYGNC NTKCQTPMGA 300
INSSMPFHNI HPLTIGECPK YVKSNRLVLA TGLRNSPQRE RRRKKRGLFG AIAGFIEGGW 360
QGMVDGWYGY HHSNEQGSGY AADKESTQKA IDGVTNKVNS IIDKMNTQFE AVGREFNNLE 420
RRIENLNKKM EDGFLDVWTY NAELLVLMEN ERTLDFHDSN VKNLYDKVRL QLRDNAKELG 480
NGCFEFYHKC DNECMESVRN GTYDYPQYSE EARLKREEIS GVKLESIGIY QILSIYSTVA 540

SSLALAIMVA GLSLWMCSNG SLQCRICI 568

The following known restricted T cell epitopes were used as controls in our MHC peptide binding assay to assess the binding of peptides to HLA-A*0101:

**Table 1: Sequences of control peptides used**

| SEQ ID NO: | Control Type | Peptide Sequence | ELISA (Absorption-Pass/Fail Control) |
|---|---|---|---|
| SEQ ID NO: 2 | Positive Control | YSEHPFTSQY | 0.58 |
| SEQ ID NO: 3 | Pass/Fail Control | AAGIGILTV | 0 |

The following known restricted T cell epitopes were used as controls in our MHC peptide binding assay to assess the binding of peptides to HLA-A*0201:

**Table 2: Sequences of control peptides used**

| SEQ ID NO: | Control Type | Peptide Sequence | ELISA (Absorption-Pass/Fail Control) |
|---|---|---|---|
| SEQ ID NO: 4 | Positive Control | GILGFVFTL | 1.20 |
| SEQ ID NO: 3 | Pass/Fail Control | AAGIGILTV | 0 |

The following known restricted T cell epitopes were used as controls in our MHC peptide binding assay to assess the binding of peptides to HLA-A*0301:

**Table 3: Sequences of control peptides used**

| SEQ ID NO: | Control Type | Peptide Sequence | ELISA (Absorption -Pass/Fail Control) |
|---|---|---|---|
| SEQ ID NO: 5 | Positive Control | ILRGSVAHK | 1.19 |
| SEQ ID NO: 3 | Pass/Fail Control | AAGIGILTV | 0 |

The following known restricted T cell epitopes were used as controls in our MHC peptide binding assay to assess the binding of peptides to HLA-B*0702:

**Table 4: Sequences of control peptides used**

| SEQ ID NO: | Control Type | Peptide Sequence | ELISA (Absorption -Pass/Fail Control) |
|---|---|---|---|
| SEQ ID NO: 6 | Positive Control | TPRVTGGGAM | 0.88 |
| SEQ ID NO: 3 | Pass/Fail Control | AAGIGILTV | 0 |

The following known T cell epitopes were used as controls in our MHC peptide binding assay to assess the binding of peptides to HLA-A*0801:

**Table 5: Sequences of control peptides used**

| SEQ ID NO: | Control Type | Peptide Sequence | ELISA (Absorption-Pass/Fail Control) |
|---|---|---|---|
| SEQ ID NO: 7 | Positive Control | RAKFKQLL | 0.73 |
| SEQ ID NO: 3 | Pass/Fail Control | AAGIGILTV | 0 |

The following known restricted T cell epitopes were used as controls in our MHC peptide binding assay to assess the binding of peptides to HLA-B*3501:

**Table 6: Sequences of control peptides used**

| SEQ ID NO: | Control Type | Peptide Sequence | ELISA (Absorption -Pass/Fail Control) |
|---|---|---|---|
| SEQ ID NO: 8 | Positive Control | HPVGEADYFEY | 0.55 |
| SEQ ID NO: 3 | Pass/Fail Control | AAGIGILTV | 0 |

We have found that 2 9-mer peptides from the H5 protein in H5N1 (SEQ ID NO: 1) bind with an efficiency to HLA-A*0101 that is similar as or better than the known T cell epitope that has been used as a pass/fail control SEQ ID NO: 3.
These peptides of the invention are listed in Table 7 below and are listed in single letter amino acid nomenclature:

**Table 7: Sequences of peptides of the invention binding to HLA-A*0101**

| SEQ ID NO: | Position of first amino acid in H5 protein (SEQ ID NO: 1) | Peptide Sequence | ELISA (Absorption -Pass/Fail Control) |
|---|---|---|---|
| SEQ ID NO: 9 | 15 | KSDQICIGY | 0.47 |
| SEQ ID NO: 10 | 86 | FINVPEWSY | 0.16 |

Further we have found that 16 9-mer peptides from the H5 protein in H5N1 (SEQ ID NO: 1) bind with an efficiency to HLA-A*0201 that is similar as or better than a known T cell epitope that has been used as a pass/fail control.
These peptides of the invention are listed in Table 8 below and are listed in single letter amino acid nomenclature:

**Table 8: Sequences of peptides of the invention binding to HLA-A*0201**

| SEQ ID NO: | Position of first amino acid in H5 protein (SEQ ID NO: 1) | Peptide Sequence | ELISA (Absorption -Pass/Fail Control) |
|---|---|---|---|
| SEQ ID NO: 11 | 5 | VLLFAIVSL | 0.47 |
| SEQ ID NO: 12 | 6 | LLFAIVSLV | 1.06 |
| SEQ ID NO: 13 | 8 | FAIVSLVKS | 0.07 |
| SEQ ID NO: 14 | 9 | AIVSLVKSD | 0.43 |
| SEQ ID NO: 15 | 10 | IVSLVKSDQ | 0.04 |
| SEQ ID NO: 16 | 12 | SLVKSDQIC | 0.09 |
| SEQ ID NO: 17 | 24 | HANNSTEQV | 0.43 |
| SEQ ID NO: 18 | 34 | TIMEKNVTV | 0.99 |
| SEQ ID NO: 19 | 35 | IMEKNVTVT | 0.02 |
| SEQ ID NO: 20 | 36 | MEKNVTVTH | -0.02 |
| SEQ ID NO: 21 | 45 | AQDILEKKH | 0.15 |
| SEQ ID NO: 22 | 56 | KLCDLDGVK | 0.20 |
| SEQ ID NO: 23 | 66 | LILRDCSVA | 0.57 |
| SEQ ID NO: 24 | 81 | PMCDEFINV | 0.98 |
| SEQ ID NO: 25 | 88 | NVPEWSYIV | 1.03 |
| SEQ ID NO: 26 | 94 | YIVEKANPV | 1.02 |

Further we have found 4 9-mer peptides from the H5 protein, SEQ ID NO: 1, which bind with an efficiency to HLA-A*0301 that is similar as or better than a known T cell epitope that has been used as a pass/fail control. These peptides of the invention are listed in Table 9 below and are listed in single letter amino acid nomenclature:

**Table 9: Sequences of peptides of the invention binding to HLA-A*0301**

| SEQ ID NO: | Position of first amino acid in H5 protein (SEQ ID NO: 1) | Peptide Sequence | ELISA (Absorption -Pass/Fail Control) |
|---|---|---|---|
| SEQ ID NO: 9 | 15 | KSDQICIGY | 0.05 |
| SEQ ID NO: 27 | 39 | NVTVTHAQD | -0.04 |
| SEQ ID NO: 28 | 48 | ILEKKHNGK | -0.01 |
| SEQ ID NO: 22 | 56 | KLCDLDGVK | 1.16 |

Further we have found 4 9-mer peptides from the H5 protein, SEQ ID NO: 1, which bind with an efficiency to HLA-B*0702 that is similar as or better than that of a known T cell epitope that has been used as a pass/fail control. These peptides of the invention are listed in Table 10 below and are listed in single letter amino acid nomenclature:

**Table 10: Sequences of peptides binding to HLA-B*0702**

| SEQ ID NO: | Position of first amino acid in H5 protein (SEQ ID NO: 1) | Peptide Sequence | ELISA (Absorption -Pass/Fail Control) |
|---|---|---|---|
| SEQ ID NO: 29 | 13 | LVKSDQICI | -0.06 |
| SEQ ID NO: 18 | 34 | TIMEKNVTV | 0.00 |
| SEQ ID NO: 30 | 67 | ILRDCSVAG | 0.03 |
| SEQ ID NO: 26 | 94 | YIVEKANPV | 0.11 |

Further we have found 14 9-mer peptides from the H5 protein, SEQ ID NO: 1, which bind with an efficiency to HLA-B*0801 that is similar as or better than the known T cell epitope that has been used as a pass/fail control SEQ ID NO: 3. These peptides of the invention are listed in Table 11 below and are listed in single letter amino acid nomenclature:

**Table 11: Sequences of peptides of the invention binding to HLA-A*0801**

| SEQ ID NO: | Position of first amino acid in H5 protein (SEQ ID NO: 1) | Peptide Sequence | ELISA (Absorption -Pass/Fail Control) |
|---|---|---|---|
| SEQ ID NO: 31 | 11 | VSLVKSDQI | 0.36 |
| SEQ ID NO: 32 | 33 | DTIMEKNVT | 0.08 |
| SEQ ID NO: 18 | 34 | TIMEKNVTV | 0.80 |
| SEQ ID NO: 27 | 39 | NVTVTHAQD | 0.08 |
| SEQ ID NO: 33 | 42 | VTHAQDILE | 0.31 |
| SEQ ID NO: 34 | 47 | DILEKKHNG | 0.53 |
| SEQ ID NO: 28 | 48 | ILEKKHNGK | 0.95 |
| SEQ ID NO: 35 | 64 | KPLILRDCS | 0.10 |
| SEQ ID NO: 36 | 65 | PLILRDCSV | 0.65 |
| SEQ ID NO: 23 | 66 | LILRDCSVA | 0.07 |
| SEQ ID NO: 37 | 73 | VAGWLLGNP | 0.04 |
| SEQ ID NO: 38 | 76 | WLLGNPMCD | 0.03 |
| SEQ ID NO: 39 | 79 | GNPMCDEFI | 0.04 |
| SEQ ID NO: 26 | 94 | YIVEKANPV | 0.32 |

Further we have found that 8 9-mer peptides from the H5 protein in H5N1 (SEQ ID NO: 1) bind with an efficiency to HLA-B*3501 that is similar as or better than the known T cell epitope that has been used as a pass/fail control SEQ ID NO: 3. These peptides of the invention are listed in Table 12 below and are listed in single letter amino acid nomenclature:

**Table 12: Sequences of peptides of the invention binding to HLA-B*3501**

| SEQ ID NO: | Position of first amino acid in H5 protein (SEQ ID NO: 1) | Peptide Sequence | ELISA (Absorption -Pass/Fail Control) |
|---|---|---|---|
| SEQ ID NO: 40 | 7 | LFAIVSLVK | 0.10 |
| SEQ ID NO: 16 | 12 | SLVKSDQIC | -0.03 |

| | | | |
|---|---|---|---|
| SEQ ID NO: 29 | 13 | LVKSDQICI | 0.04 |
| SEQ ID NO: 17 | 24 | HANNSTEQV | 0.43 |
| SEQ ID NO: 41 | 51 | KKHNGKLCD | -0.05 |
| SEQ ID NO: 42 | 74 | AGWLLGNPM | -0.06 |
| SEQ ID NO: 43 | 78 | LGNPMCDEF | 0.35 |
| SEQ ID NO: 10 | 86 | FINVPEWSY | 0.57 |

In its first aspect the invention therefore concerns one or more of the isolated peptides selected from the group consisting of:

| | |
|---|---|
| KSDQICIGY | (SEQ ID NO: 9), |
| FINVPEWSY | (SEQ ID NO: 10), |
| VLLFAIVSL | (SEQ ID NO: 11), |
| LLFAIVSLV | (SEQ ID NO: 12), |
| FAIVSLVKS | (SEQ ID NO: 13), |
| AIVSLVKSD | (SEQ ID NO: 14), |
| IVSLVKSDQ | (SEQ ID NO: 15), |
| SLVKSDQIC | (SEQ ID NO: 16), |
| HANNSTEQV | (SEQ ID NO: 17), |
| TIMEKNVTV | (SEQ ID NO: 18), |
| IMEKNVTVT | (SEQ ID NO: 19), |
| MEKNVTVTH | (SEQ ID NO: 20), |
| AQDILEKKH | (SEQ ID NO: 21), |
| KLCDLDGVK | (SEQ ID NO: 22), |
| LILRDCSVA | (SEQ ID NO: 23), |
| PMCDEFINV | (SEQ ID NO: 24), |
| NVPEWSYIV | (SEQ ID NO: 25), |
| YIVEKANPV | (SEQ ID NO: 26), |
| NVTVTHAQD | (SEQ ID NO: 27), |
| ILEKKHNGK | (SEQ ID NO: 28), |
| LVKSDQICI | (SEQ ID NO: 29), |
| ILRDCSVAG | (SEQ ID NO: 30), |
| VSLVKSDQI | (SEQ ID NO: 31), |
| DTIMEKNVT | (SEQ ID NO: 32), |
| VTHAQDILE | (SEQ ID NO: 33), |
| DILEKKHNG | (SEQ ID NO: 34), |
| KPLILRDCS | (SEQ ID NO: 35), |
| PLILRDCSV | (SEQ ID NO: 36), |
| VAGWLLGNP | (SEQ ID NO: 37), |
| WLLGNPMCD | (SEQ ID NO: 38), |
| GNPMCDEFI | (SEQ ID NO: 39), |
| LFAIVSLVK | (SEQ ID NO: 40), |
| KKHNGKLCD | (SEQ ID NO: 41), |
| AGWLLGNPM | (SEQ ID NO: 42), and |
| LGNPMCDEF | (SEQ ID NO: 43). |

The binding assay was carried out according the methods described in the applicant's co-pending British patent application GB0519029.3, measuring the extent of assembly of the peptides at a known concentration with the HLA alpha and beta chains over 24 hours. The extent of assembly was measured quantitatively for each peptide using a solid phase binding assay (ELISA) with a conformational antibody that only detects fully assembled MHC complexes. The ELISA absorption readings were taken for all peptides to be tested and the respective control peptides, which are known T cell epitopes, i.e. they are known to elicit T cell immune responses in humans. SEQ ID NO: 2 is a peptide binding with high efficiency to HLA-A*0101, SEQ ID NO: 4 is a peptide binding with high efficiency to HLA-A*0201, SEQ ID NO: 5 is a peptide binding with high efficiency to HLA-A*0301, SEQ ID NO: 6 is a peptide binding with high efficiency to HLA-B*0702, SEQ ID NO: 7 is a peptide binding with high efficiency to HLA-B*0801, SEQ ID NO: 8 is a peptide binding with high efficiency to HLA-B*3501, and SEQ ID NO: 3 is a peptide binding with intermediate efficiency to HLA-A*0201 which is used as a pass/fail control in all assays. SEQ ID NO: 3 was chosen as a pass/fail control since it is a relatively weak binding peptide on the one hand, but it has still been confirmed as a T cell epitope on the other. The extent of the binding of SEQ ID NO: 3 was always assessed for its binding to HLA-A*0201. The peptides of the invention were selected based on their ability to bind with similar or better efficiency to the MHC alleles as stated in the tables above than the control peptide is able to bind to HLA-A*0201. Although binding of the pass/fail control peptide was therefore measured with a different MHC allele than for the peptides to be tested in the assay, the use of the pass/fail control peptide for this purpose was validated: the same molar amounts were used for each component in the assay irrespective of the MHC allele studied and it was ascertained that the detection system renders substantially identical conformational signals when identical molar amounts of purified conformational MHC complexes are used for each of HLA-A*0101, HLA-A*0201, HLA-A*0301, HLA-B*0702, HLA-B*0801, and HLA-B*3501. The tables above show the ELISA absorption readings taken for each peptide with the absorption reading for the control peptide subtracted.

### The peptides of the invention are useful in a number of ways:

They may be used as isolated peptides, e.g. in the form of synthetic peptides in prophylactic or therapeutic vaccines to treat or vaccinate against influenza infection. They are useful in this way when bound to HLA-A1, A2, A3, B7, B8, and HLA-B35 complexes, as applicable, and on their own. Alternatively they may be segments in larger protein based vaccine constructs. They may be administered in the form of one or more of the isolated peptides of the invention or lipopeptide vaccine, optionally with an adjuvant such as described in US6,733,973.

Obtaining the isolated peptides of the invention is well known in the art, e.g. by solid phase peptide synthesis, which is e.g. offered as a routine service to researchers by many companies today, such as Sigma-Genosys, Woodlands, Texas, USA.

The invention further concerns DNA or RNA sequences encoding one or more of the peptides of the invention. These DNA or RNA sequences may be used in DNA or RNA constructs for heterologous expression in a prokaryotic host cell, such as *E.coli.,* or a eukaryotic host cell, such as an insect cell or mammalian cell. The constructs may be viral vector based constructs such as vaccinia or pox vector constructs, which may be used as vaccines or other immunotherapies to treat or vaccinate against influenza infection. The actual sequences of such coding DNA or RNA sequences may be based on the original coding sequences of the H5N1 virus studied here and may be adapted based on the host cell in which these sequences are to be expressed. Back-translation from protein to DNA and RNA coding sequences is documented extensively in the literature. Such coding DNA or RNA sequences may offer additional design options, such as selecting codon-optimised sequences for the expression in the host of interest or other codon choices, e.g. suited to the restriction sites in the expression system used.

In a third aspect thereof the invention therefore concerns a DNA or RNA sequence encoding one or more of the peptides of the invention and a vector comprising such a DNA or RNA sequence.

The construction of vaccines based on the peptides of the invention or RNA, DNA coding therefore is described in some detail in US6,733,973.

In another alternative embodiment the peptides may be incorporated in an oligomeric MHC-peptide complex, such as in fluorescent labelled MHC pentamers as described in WO04018520, which is incorporated herein by reference, or MHC tetramers which may be formed by coupling monomeric MHC-peptide complexes incorporating the peptides of the invention to a multivalent entity at a specific attachment site, as described in US 5,635,363, which are useful for labelling, detecting and isolating antigen-specific T cells. Such MHC multimers are consequently useful for immune monitoring of single-antigen specific T cells that are reactive to the peptides of the invention in patients.

In a fourth aspect therefore the invention concerns an MHC-peptide complex comprising one or more of the peptides of the invention and in a fifth aspect the invention concerns an oligomeric MHC-peptide complex comprising one or more of the peptides of the invention.

In yet another embodiment the binding peptides of the invention can be used to raise polyclonal or monoclonal antibodies against the peptides of the invention, which may be useful for diagnosing influenza infection or presentation of the specific peptide on a cell. The detection of such specific peptide presentation can be very important when tracking the distribution of MHC complexes presenting a particular peptide *in vivo,* e.g. in the context of a vaccination protocol where the vaccination occurs through a particular administration route and/or location and is designed to effect presentation of a specific peptide. In this case it is often required that the MHC-peptide presentation is then translated to a specific site of action in the vaccinated individual's tissues. Monitoring the effectiveness of such translocation of antigen presentation is possible by using binding of antibodies that are specific to the MHC-peptide combination of interest, whereby the antibodies themselves are labelled to enable visualisation, e.g. through a fluorescent or radioactive label, which may be traceable *in vivo.* Further such antibodies may also be used in antibody-based therapy to fight influenza infection, e.g. as normal complement-fixing antibodies or radiolabelled antibodies that are used to kill infected cell with an effective dose of radiation. The production of radiolabelled monoclonal antibodies for treating cancer in a similar way is described in detail in WO9300927, which is incorporated herein by reference. Although W09300927 describes the use of radiolabelled monoclonal antibodies for treating cancer the methodology is readily translated to the treatment of infectious diseases.

In a sixth aspect the invention therefore comprises an antibody specific to one or more of the peptides of the invention or specific to an MHC-peptide complex comprising one or more of the peptides of the invention.

In a further embodiment of the invention cytolytic T cell lines or clones that are reactive to the peptides of the invention may be raised. Raising such T cell lines may be effected as described in US 5,585,461 or US6,733,973. Such antigen-specific T cell lines can also be generated by incorporating the peptides of the invention in MHC pentamers, such as described in the applicant's WO04018520. Such MHC pentamers can be coupled to paramagnetic beads, such as Dynabeads®, which are useful for isolating peptide-specific T cells. Cells isolated in this manner can be expanded in culture and are suitable for the therapeutic protocols described in. Such cell lines are further useful as positive controls for monitoring immune responses against the peptides of interest in patients. Alternatively they may be useful in a therapeutic application where it is desirable to administer T cells specific to one or more peptides of the invention to a patient either autologously or allogeneically, e.g. in order to increase their immune response to the antigen in question, such as described in US6,733,973.

In a seventh aspect the invention therefore concerns a cytolytic T cell line or clone specific to one or more of the peptides of the invention.

Alternatively the peptides of the invention may be administered in the form of a cellular vaccine through the administration of autologous or allogeneic antigen presenting cells or dendritic cells that have been treated *in vitro* so as to present one or more of the peptides of the invention on their surface. The preparation of such antigen presenting cells is described in US6,733,973.

In an eighth aspect the invention therefore concerns an isolated dendritic cell presenting one or more of the peptides of the invention.

In a ninth aspect the invention concerns pharmaceutical composition comprising one or more from the group consisting of one or more of the peptides of the invention, a DNA or RNA sequence of the invention, a vector of the invention, an MHC-peptide complex of the invention, an oligomeric MHC-peptide complex the invention, an antibody of the invention, a cytolytic T cell line of the invention, a dendritic cell of the invention.

The pharmaceutical composition of the invention can be used for treating influenza infection or be administered for prophylactic vaccination.

Pharmaceutical compositions comprising the peptides are useful for, e.g. parenteral administration, i.e. subcutaneously, intramuscularly or intravenously, but not limited thereto.

The compositions for parenteral administration may for example comprise the active ingredients dissolved or suspended in an acceptable carrier, preferably an aqueous carrier. A variety of aqueous carriers can be used, e.g. buffered water, 0.4% saline, 0.3% glycine and the like. These solutions are sterile and generally free of particulate matter and may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. The concentration of the active ingredients in these formulations can vary widely, i.e. in the case of soluble matter from less than about 1 pg/ml, usually at least about 0.1 mg/ml to as much as 10 - 100 mg/ml and will be selected primarily based on fluid volumes, viscosities, etc. in accordance with the particular mode of administration selected.

In a tenth aspect the invention concerns a diagnostic composition comprising a label and one or more from the group consisting of one or more of the peptides of the invention, a DNA or RNA sequence of the invention, a vector of the invention, an MHC-peptide complex of the invention, an oligomeric MHC-peptide complex the invention, an antibody of the invention, a cytolytic T cell line of the invention, a dendritic cell of the invention.

The diagnostic compositions of the invention can be used to detect, characterise and monitor influenza infections in individuals, detect cells that are presenting or are reactive to the peptides of the invention in a cell population and generally assess the immune competence of individuals relative to influenza infection. For this purpose such reagents may be provided with a label, such as a radioactive or fluorescent label as described in US6,733,973 and WO04018520.

It will be understood that the foregoing examples are merely given to illustrate the invention and the invention is not limited thereto. Other uses of the peptide sequences described herein will be evident to the skilled practitioner.

## Claims

1. An isolated peptide selected from the group consisting of:
| | |
|---|---|
| KSDQICIGY | (SEQ ID NO: 9), |
| FINVPEWSY | (SEQ ID NO: 10), |
| VLLFAIVSL | (SEQ ID NO: 11), |
| LLFAIVSLV | (SEQ ID NO: 12), |
| FAIVSLVKS | (SEQ ID NO: 13), |
| AIVSLVKSD | (SEQ ID NO: 14), |
| IVSLVKSDQ | (SEQ ID NO: 15), |
| SLVKSDQIC | (SEQ ID NO: 16), |
| HANNSTEQV | (SEQ ID NO: 17), |
| TIMEKNVTV | (SEQ ID NO: 18), |
| IMEKNVTVT | (SEQ ID NO: 19), |
| MEKNVTVTH | (SEQ ID NO: 20), |
| AQDILEKKH | (SEQ ID NO: 21), |
| KLCDLDGVK | (SEQ ID NO: 22), |
| LILRDCSVA | (SEQ ID NO: 23), |
| PMCDEFINV | (SEQ ID NO: 24), |
| NVPEWSYIV | (SEQ ID NO: 25), |
| YIVEKANPV | (SEQ ID NO: 26), |
| NVTVTHAQD | (SEQ ID NO: 27), |
| ILEKKHNGK | (SEQ ID NO: 28), |
| LVKSDQICI | (SEQ ID NO: 29), |
| ILRDCSVAG | (SEQ ID NO: 30), |
| VSLVKSDQI | (SEQ ID NO: 31), |
| DTIMEKNVT | (SEQ ID NO: 32), |
| VTHAQDILE | (SEQ ID NO: 33), |
| DILEKKHNG | (SEQ ID NO: 34), |
| KPLILRDCS | (SEQ ID NO: 35), |
| PLILRDCSV | (SEQ ID NO: 36), |
| VAGWLLGNP | (SEQ ID NO: 37), |
| WLLGNPMCD | (SEQ ID NO: 38), |
| GNPMCDEFI | (SEQ ID NO: 39), |
| LFAIVSLVK | (SEQ ID NO: 40), |
| KKHNGKLCD | (SEQ ID NO: 41), |
| AGWLLGNPM | (SEQ ID NO: 42), and |
| LGNPMCDBF | (SEQ ID NO: 43). |

2. A DNA or RNA sequence encoding one or more of the peptides of claim 1.

3. A vector comprising a DNA or RNA sequence of claim 2.

4. An MHC-peptide complex comprising one or more of the peptides of claim 1.

5. An oligomeric MHC-peptide complex comprising one or more of the peptides of claim 1.

6. An antibody specific to one or more of the peptides of claim 1 or an MHC-peptide complex comprising one or more of the peptides of claim 1.

7. A cytolytic T cell line or clone specific to one or more of the peptides of claim 1.

8. A dendritic cell primed with one or more of the peptide of claims 1.

9. A pharmaceutical composition comprising one or more from the group consisting of one or more of the peptides of claims 1, a DNA or RNA sequence of claim 2, a vector of claim 3, an MHC-peptide complex of claim 4, an oligomeric MHC-peptide complex of claim 5, an antibody of claim 6, a cytolytic T cell line or clone of claim 7, a dendritic cell of claim 8.

10. A diagnostic composition comprising a label and one or more from the group consisting of one or more of the peptides of claims 1, a DNA or RNA sequence of claim 2, a vector of claim 3, an MHC-peptide complex of claim 4, an oligomeric MHC-peptide complex of claim 5, an antibody of claim 6, a cytolytic T cell line or clone of claim 7, a dendritic cell of claim 8.
